(19) Europäisches Patentamt
European Patent Office
Office européen des brevets

(11) **EP 3 024 339 B1**

(12) **EUROPEAN PATENT SPECIFICATION**

(45) Date of publication and mention of the grant of the patent:
**22.03.2017 Bulletin 2017/12**

(21) Application number: **14732535.1**

(22) Date of filing: **17.06.2014**

(51) Int Cl.:
*A23L 33/16* (2016.01)   *A23L 33/17* (2016.01)
*A23L 33/00* (2016.01)   *A61K 31/295* (2006.01)
*A61K 38/40* (2006.01)   *A61K 38/00* (2006.01)
*A23C 9/13* (2006.01)   *A23C 9/133* (2006.01)
*A61K 38/19* (2006.01)   *A61K 33/26* (2006.01)
*A23L 19/00* (2016.01)   *A23L 33/165* (2016.01)
*A23L 33/19* (2016.01)

(86) International application number:
**PCT/EP2014/062670**

(87) International publication number:
**WO 2015/000694 (08.01.2015 Gazette 2015/01)**

(54) **LACTOFERRIN-OSTEOPONTIN-IRON COMPLEX**

LACTOFERRIN-OSTEOPONTIN-EISENKOMPLEX

COMPLEXE LACTOFERRINE-OSTÉOPONTINE-FER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.07.2013 EP 13175395**

(43) Date of publication of application:
**01.06.2016 Bulletin 2016/22**

(73) Proprietor: **Nestec S.A.**
**1800 Vevey (CH)**

(72) Inventors:
• **KASTENMAYER, Peter**
  **CH-1800 Vevey (CH)**
• **KOCHHAR, Sunil**
  **CH-1073 Savigny (CH)**
• **REZZI, Serge André Dominique**
  **CH-1623 Semsales (CH)**

(74) Representative: **Couzens, Patrick John**
**Nestec S.A.**
**Centre de Recherche Nestlé**
**Vers-chez-les-Blanc**
**Case Postale 44**
**1000 Lausanne 26 (CH)**

(56) References cited:
**WO-A1-2004/060392**

• **AARON P. YAMNIUK ET AL: "Thermodynamic characterization of the interactions between the immunoregulatory proteins osteopontin and lactoferrin", MOLECULAR IMMUNOLOGY, vol. 46, no. 11-12, 1 July 2009 (2009-07-01), pages 2395-2402, XP055087676, ISSN: 0161-5890, DOI: 10.1016/j.molimm.2009.04.024**

**Description**

The present invention relates to complexes of lactoferrin, osteopontin and iron. In particular the invention relates to a composition comprising at least 2 g/kg of lactoferrin-osteopontin-iron complex for use in the treatment or prevention of iron deficiency. An aspect of the invention is a fortified foodstuff comprising a lactoferrin-osteopontin-iron complex. Further aspects of the invention are a process for fortifying a food product with iron and the use of a lactoferrin-osteopontin-iron complex to fortify a food product with iron.

Billions of people around the world suffer from 'hidden hunger' or micronutrient malnutrition. They do not get enough of the micronutrients required to lead healthy productive lives from the foods that they eat. Micronutrients are vitamins and minerals (such as vitamin A, zinc, and iron) and are absolutely essential to good health. Micronutrient malnutrition can lower IQ, cause stunting and blindness in children, lower resistance to disease in both children and adults, and increase risks for both mothers and infants during childbirth. Iron deficiency is the most common and widespread nutritional disorder in the world [J. Umbreit, American Journal of Hematology 78, 225 (2005)]. As well as affecting a large number of children and women in developing countries, it is the only nutrient deficiency which is also significantly prevalent in industrialized countries. In wealthier countries people may voluntarily choose a diet which may cause a reduced iron intake, such as a vegetarian diet. Infants and small children need more iron because they are growing and so an inadequate diet may lead to iron deficiency. Pregnant women are at risk of iron deficiency because the growing fetus requires large amounts of iron.

When iron reserves in the body are exhausted, anemia develops. Anemia causes paleness, weakness, and fatigue. Less than 20% of iron in a typical diet is absorbed into the body. Thus, most people with iron deficiency need to take iron supplements by mouth usually once or twice a day. Iron can be supplemented by the oral route using various pharmacological forms, such as ferrous sulphate, and in complexes with gluconate, dextran, carbonyl iron, and other salts. Sometimes ascorbic acid is added for better absorption. Many iron salts, such as ferrous sulphate and ferrous citrate, have an unpleasant taste. Patients are often advised to take iron in supplements on an empty stomach because some components of foods (such as vegetable fibres and phytates) will complex free iron and reduce its absorption. However, taking iron supplements on an empty stomach can cause indigestion and constipation. It would be desirable to find further forms of iron which can be used to treat and prevent iron deficiency, especially forms of iron which have a neutral taste and remain effective when combined with food.

The World Health Organization (WHO) recommended that ferrous sulphate should be the first choice among iron fortificants because of its high bioavailability [Guidelines on food fortification with micronutrients. World Health Organization, 2006]. However, potentially adverse organoleptic changes can occur in certain food products fortified with ferrous sulphate that necessitate the choice of other iron forms that are less chemically reactive in that food matrix, although at the cost of lower bioavailability relative to ferrous sulphate. For example iron can accelerate oxidation reactions, adversely altering the food's flavour, and iron can also lead to colour changes. Free soluble iron forms complexes with coloured compounds in the food causing a colour change. This problem typically occurs after prolonged storage, on cooking the food, or if the food is sterilized using heat. Foods may need to be sterilized to provide a long shelf life or to be safely consumed by sensitive groups such as the sick or very young. The problem of colour change when fortifying food with iron is particularly apparent with food compositions containing fruit. The colour of many fruits is derived from compounds such as anthocyanins which may change colour in the presence of iron. The iron forms complexes with the anthocyanin molecules. This causes a change in the wavelength of light absorbed by the anthocyanins (a bathochromic shift) leading to an undesirable colour change. Fruit intrinsically provides a good source of beneficial dietary nutrients, and so is a good basis for delivering additional nutritional benefits to food. There is therefore a need to provide iron fortified foodstuffs which do not exhibit undesirable colour changes, for example foodstuffs comprising fruit.

EP1011344 describes chocolate-flavoured beverage mixes and other edible mixes that are fortified with sources of iron such as ferrous fumarate and ferrous sulphate, yet do not develop undesirable gray colour when the beverage mix is reconstituted with aqueous liquids including fruit juice. The problem of gray colour development is solved by including edible acids such as citric or malic acid as buffering agents in the beverage mix so that the pH of the reconstituted chocolate beverage is about 6.5 or less. Controlling the pH to be acidic does not always suit the desired taste of the product. Also, controlling the pH is generally most suitable for beverages, where any coloured components are generally dissolved or dispersed in a continuous aqueous phase and so can be influenced by added acids. For non-beverage food compositions it may be difficult to ensure that all the components responsible for the development of an undesirable colour are affected by added acids.

WO97/15201 discloses colour-stable iron fortified fruit flavoured dry drink mixes where the iron is added as ferric sulphate encapsulated in solid fats, or as iron chelated with amino acids. However, it may not always be desirable to add solid fats into food; and iron chelated with amino acids is more expensive than many other iron compounds.

EP1040766 describes iron-enriched beverages comprising iron and a lactoferrin, with at least one acidulant selected from acetic acid, gluconic acid and lactic acid. This combination of ingredients was found to avoid an unpleasant taste of iron.

WO2005/051088 describes an iron-containing human milk fortifier where the antimicrobial properties of the milk are not significantly inhibited. This was achieved by having little or no soluble unbound iron. The addition of lactoferrin was found to be helpful in reducing soluble unbound iron fractions.

WO2004/060392 describes therapeutic and nutritional compositions comprising lactoferrin proteins complexed with one or more iron atoms.

There remains a need to find further forms of iron which can be used to treat and prevent iron deficiency without an unpleasant taste and which do not cause undesirable changes of colour when combined with food. In particular there is a need to provide forms of iron which are less chemically reactive in a food matrix than ferrous sulphate, but which have similar or improved bioavailability. In addition there is a need to provide improved or alternative fortified foodstuffs which provide iron with good bioavailability, stabilized against colour change by ingredients from natural sources.

An object of the present invention is to improve the state of the art and to provide an improved solution to overcome at least some of the inconveniences described above, or at least to provide a useful alternative. Any reference to prior art documents in this specification is not to be considered an admission that such prior art is widely known or forms part of the common general knowledge in the field. As used in this specification, the words "comprises", "comprising", and similar words, are not to be interpreted in an exclusive or exhaustive sense. In other words, they are intended to mean "including, but not limited to".

The object of the present invention is achieved by the subject matter of the independent claims. The dependent claims further develop the idea of the present invention.

Accordingly, the present invention provides in a first aspect a composition comprising at least 2 g/kg of lactoferrin-osteopontin-iron complex for use in the treatment or prevention of iron deficiency. In a second aspect, the invention provides a fortified foodstuff comprising at least 2 g/kg of lactoferrin-osteopontin-iron complex. Further aspects of the invention relate to a process for fortifying a food product with iron and the use of a lactoferrin-osteopontin-iron complex to fortify a food product with iron.

Lactoferrin, also known as lactotransferrin, is an 80 kDa globular glycoprotein. Lactoferrin occurs in human milk at about 1500 mg/L and in bovine milk at about 150 mg/L, it may also be found in many mucosal secretions such as tears and saliva. Lactoferrin is part of the innate immune system of the body and has a large number of biological activities including antibacterial, antiviral, antifungal, antitumour and anti-inflammatory activities. Osteopontin is a 36 kDa, anionic, predominantly unstructured phosphorylated glycoprotein. Osteopontin occurs in human milk at about 150 mg/L and bovine milk at about 15 mg/L.

Lactoferrin (LF) and osteopontin (OPN) have been shown to interact with each other through a complex mechanism involving 1, 2 or 3 LF molecules binding to a single molecule of OPN [A.P.Yamniuk et al., Molecular Immunology, 46, 2395 (2009)].

The inventors surprisingly found that a lactoferrin-osteopontin-iron complex provided a bio-accessible source of iron having good colour stability properties when combined with materials such as food. For example, a strawberry and banana yoghurt fortified with a lactoferrin-osteopontin-iron complex showed less colour change after heat treatment than yoghurts fortified at the same iron level using NaFeEDTA, ferrous sulphate ($FeSO_4$) or a lactoferrin-iron complex. Ferrous sulphate is known to provide a highly bioavailable source of iron and so the inventors were surprised to find that the bio-accessibility of the iron in yoghurt fortified with lactoferrin-osteopontin-iron complex was similar or even higher than the yoghurt fortified with ferrous sulphate at the same level of iron.

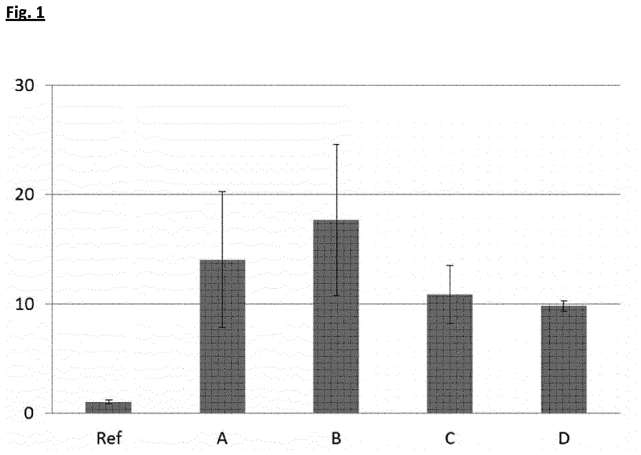

Figure 1 shows iron bioacessability measured by in vitro digestion/Caco-2 cells. Ferritin level in ng/mg protein $\pm$ SEM or Stdev (n=9) is plotted for Jogolino samples fortified with different iron containing compounds at a level of 100 $\mu$g/g of iron: Ref = unfortified, A = $FeSO_4$, B = lactoferrin-osteopontin-iron, C = lactoferrin-iron and D = NaFeEDTA

Consequently the present invention relates in part to a composition comprising at least 2 g/kg of lactoferrin-osteopontin-iron complex for use in the treatment or prevention of iron deficiency. The level of at least 2 g/kg of lactoferrin-osteopontin-iron complex provides an effective level of iron for treatment or prevention of iron deficiency. The level of 2 g/kg is nearly twice the level of lactoferrin-osteopontin-iron complex found in human milk. In human milk, LF is in a tenfold excess of OPN by weight. Taking the mass of LF as 80 kDa and the mass of OPN as 36 kDa [A.P.Yamniuk et al., Molecular Immunology, 46, 2395 (2009)] this is 4.5 times as many molecules of LF as OPN. Yamniuk et al. have shown that there is a maximum molar ratio of 3:1 LF:OPN in the LF-OPN-iron complex. Accordingly, with all the OPN complexed with LF, and a level of 150 mg/kg of OPN in human milk, there will be a maximum of 1.15 g/kg of LF-OPN-iron complex in human milk.

The composition of the invention may comprise at least 4 g/kg of lactoferrin-osteopontin-iron complex, for example at least 8 g/kg of lactoferrin-osteopontin-iron complex. The composition of the invention may be predominantly the lactoferrin-osteopontin-iron complex, such as a composition which comprises at least 600 g/kg of lactoferrin-osteopontin-iron complex, for example a compressed tablet containing 60% lactoferrin-osteopontin-iron complex and 40 % of other materials such as dry binders. The composition for use in the treatment or prevention of iron deficiency may consist of the lactoferrin-osteopontin-iron complex. For example, a powdered lactoferrin-osteopontin-iron complex may be orally administered to a subject.

In the lactoferrin-osteopontin-iron complex, lactoferrin is bound to osteopontin, for example by electrostatic interaction. The iron present in the complex may be bound to lactoferrin and/or osteopontin. Iron may be present as $Fe^{2+}$ or $Fe^{3+}$ ions. The ratio of LF:OPN in the complex may vary depending on its preparation, for example it may vary according to the amount of LF and OPN which are combined to form the complex. As shown by Yamniuk *et al.* the ratio of lactoferrin to osteopontin in each complex may be 3:1, 2:1 or 1:1 on a molar basis, which is approximately 6.6:1, 4.4:1 and 2.2:1 by weight. Intermediate values may be possible in the overall bulk ratio, due to a combination of different ratios at a molecular level. The ratio of lactoferrin to osteopontin in the complex used in the invention may be between 6.6:1 and 2.2:1 by weight, for example between 4.4:1 and 2.2:1 by weight, for further example between 3:1 and 2.2:1 by weight. The ratio of lactoferrin to osteopontin in the complex may be about 2.2:1 by weight. The ratio of lactoferrin to osteopontin in the complex refers to the overall bulk ratio in the lactoferrin-osteopontin-iron complex.

Although the levels of lactoferrin and osteopontin in the milk of different mammalian species vary, the LF:OPN ratio is essentially the same, being about 10:1 by weight. For use in the treatment or prevention of iron deficiency it is an advantage to have lower ratios of lactoferrin to osteopontin. Increasing the amount of osteopontin relative to lactoferrin reduces the LF:OPN ratio. Osteopontin plays an important role both in bone remodelling and in the functioning of the immune system, so it is an advantage to be able to provide higher levels of osteopontin for a given weight of complex. The complex with the highest level of osteopontin is the 1:1 molar complex [A.P.Yamniuk et al., Molecular Immunology, 46, 2395 (2009)]. To promote the formation of a 1:1 molar complex, osteopontin may be advantageously combined with lactoferrin in a molar excess of osteopontin. For example, equal weights of osteopontin and lactoferrin may be combined, providing a molar ratio of LF:OPN of about 1:2.2, guaranteeing the formation of the 1:1 molar complex, with excess osteopontin remaining unbound to lactoferrin.

The present invention relates to lactoferrin and osteopontin obtainable from any source. The lactoferrin and/or osteopontin may for example be obtained from milk or whey, for example bovine milk or whey. Obtaining lactoferrin or osteopontin from bovine milk or whey has the advantage of providing natural ingredients sourced from food-grade materials which may be used in food compositions without further purification.

In an embodiment, the composition of the invention is to be administered orally or enterally. Most people and animals dislike having injections and so it is an advantage to have a composition which can be administered without an injection. The composition of the invention may be a nutritional supplement. A nutritional supplement, also known as food supplement or dietary supplement, is a preparation intended to supplement the diet and provide nutrients, such as vitamins, minerals, fibre, fatty acids, or amino acids that may be missing or may not be consumed in sufficient quantities in a person's diet. The composition may be a liquid nutritional formula to be administered enterally, e.g., in hospitals.

In an embodiment the composition is to be administered to preterm or low birth weight infants. Preterm infants are those born after less than 37 weeks gestation. Low birth weight infants are those with a birth weight of less than 2.5 kg. Preterm and low birth weight infants are at risk of exhausting their body iron stores much earlier than healthy term newborns. Infants who receive iron supplementation have improved iron stores and a lower risk of developing iron deficiency anaemia when compared with those who are un-supplemented [R.J. Mills et al., Enteral iron supplementation in preterm and low birth weight infants, Cochrane database of systematic reviews (Online) 3, pp. CD005095].

In an embodiment, the composition is to be administered to pregnant or lactating women. It is very common for women to develop iron deficiency during pregnancy. Expansion of blood volume by approximately 35 percent and growth of the fetus, placenta, and other maternal tissues increase the demand for iron threefold in the second and third trimesters. Many pregnant women require an iron supplement, particularly from the 20th week of pregnancy, although supplementation should be individualized depending on the mother's iron status. Lactating females can also be iron deficient, for example due to blood loss as a result of childbirth, or due to a large quantity of iron having been passed from the mother to her developing offspring during the third trimester. If the mother's diet is iron-poor at this time, she will experience iron-deficiency.

In a further aspect, the invention provides a fortified foodstuff comprising at least 2 g/kg of lactoferrin-osteopontin-iron complex. The inventors surprisingly found that iron, added in the form of a lactoferrin-osteopontin-iron complex, may be used to fortify food materials without causing the quality problems associated with free soluble iron, such as colour change. A fortified foodstuff is a food in which the content of one of more nutrients has been increased in the food. In certain countries' food labelling legislation a distinction is made between the terms "enriched" and "fortified". The term "enriched" referring to adding back nutrients which were once present in the foodstuff but which were eliminated or reduced during processing. In the current specification the term fortified foodstuff includes foodstuffs sometimes described as enriched in nutrients where the increase in nutrient content is for the purpose of replacing nutrients lost during processing.

The fortified foodstuff according to the invention may have a ratio of lactoferrin to osteopontin in the complex of between 6.6:1 and 2.2:1 by weight, for example between 4.4:1 and 2.2:1 by weight, for further example between 3:1 and 2.2:1 by weight. The fortified foodstuff may have a ratio of lactoferrin to osteopontin in the complex of about 2.2:1 by weight.

The fortified foodstuff according to the invention may be a dairy product; a culinary product; a food for infants;

a food for pregnant women or new mothers, a beverage; a biscuit, cake or pastry product; a dessert; a nutritional formula or a pet food product. The dairy products may be for example milk-based powders, ice creams, cheese, fermented milks, and yogurt. Yoghurt is a good source of calcium, helping to form and maintain strong bones. Yoghurt may also be fortified with other beneficial minerals such as magnesium and zinc. However, fortifying yoghurt with iron presents a problem if the yoghurt contains chromophore compounds, such as may be found in fruit yoghurts. For example, a blueberry yoghurt, coloured by the anthocyanins in blueberries, will change colour after addition of iron; the anthocyanins undergoing a bathochromic shift. Culinary products are food compositions typically prepared or used in kitchens. Culinary products which may be fortified foodstuffs according to the invention include soups, sauces, bouillon, liquid seasonings and prepared meals. Free dissolved iron may cause undesirable colour change in these products and make ingredients such as fats more susceptible to oxidation leading to off flavours. Fortifying the products with the lactoferrin-osteopontin-iron complex of the invention prevents or reduces these unwanted effects.

The fortified foodstuff of the invention may be a food for infants, a food for pregnant women or new mothers. In the scope of the present invention, infants are children under the age of 12 months. The food for infants may be a foodstuff intended for the complete or partial nutrition of infants. A new mother is someone who has given birth within the previous six months, or is breastfeeding. Infants, pregnant women and new mothers are at particular risk of having an insufficient intake of bioavailable iron in their diet and so it is an advantage that the fortified foodstuff of the invention can provide good bioavailability of iron in a foodstuff which does not have an unpleasant metallic taste, or show poor stability such as undesirable colour changes.

The fortified foodstuff may be a nutritional formula. This may, for example, be a complete nutritional formula which provides sufficient types and levels of macronutrients (protein, fats and carbohydrates) and micronutrients to be sufficient as a sole source of nutrition for the subject to which it is administered. The nutritional formula may also provide partial nutrition, to act as a supplement to the existing diet of the subject. Nutritional formulas provide the body with nutrients that it may urgently need when it is affected by iron deficiency.

The fortified foodstuff of the invention may be a pet food product. Pets such as cats and dogs may suffer from iron deficiency and so benefit from a foodstuff fortified in iron.

The fortified foodstuff of the invention may be resistant to colour change over the foodstuff's shelf-life. For example, the CIELAB ΔEab* colour difference between an iron fortified food product at the time of its manufacture and the end of its shelf-life under recommended storage conditions may be less than 3.5, for example less than 2. Shelf life is the recommended length of time that foods, beverages, and many other perishable items can be stored during which the defined quality of a specified proportion of the goods remains acceptable under expected (or specified) conditions of distribution, storage and display. Typically a "best before date" (BBD) is printed on packaged perishable foods together with recommended storage conditions. Where such a BBD is indicated, the shelf-life is the time between manufacture and the BBD. Where a BBD is not indicated, the shelf-life is the equivalent period usual for the relevant product type.

The fortified foodstuff of the invention may be resistant to colour change during heat treatment of the product. For example, the colour change of the fortified foodstuff measured as ΔEab* may be less than 3.5, for example less than 2, after a heat treatment of 2 minutes at 105 °C. Such a heat treatment is typical for packaged foodstuffs which are intended to have a long shelf-life or to be consumed by vulnerable groups, such as infants.

The CIE 1976 L*a*b* (hereinafter CIELAB) colour scale is one method of measuring colour proposed by the Commission Internationale de l'Éclairage (CIE) [CIE Technical Report, Colorimetry 2nd Edition, CIE 15.2 - 1986, corrected reprint 1996]. The CIELAB colour space is produced by plotting the quantities L*, a*, b* in rectangular coordinates. The L* coordinate of an object is the lightness intensity as measured on a scale from 0 (black) to 100 (absolute white). The a* and b* coordinates have no specific numerical limits. The parameter a* runs from pure green (negative a*) to pure red (positive a*), while b* runs from pure blue (negative b*) to pure yellow (positive b*).

In the CIELAB colour space, colour difference may be calculated as a single value taking into account the differences between the L*, a* and b* values of two samples. The colour difference ΔEab* is calculated as follows:

$$\Delta Eab^* = \sqrt{(\Delta L^*)^2 + (\Delta a^*)^2 + (\Delta b^*)^2}$$

A further aspect of the invention is a process for fortifying a food product with iron comprising forming a lactoferrin-osteopontin-iron complex by combining lactoferrin, osteopontin and iron, the lactoferrin, osteopontin and iron being in aqueous solution; preparing a food product; and adding the lactoferrin-osteopontin-iron complex to the food product. The components for forming the lactoferrin-osteopontin-iron complex may be combined in any order. The iron may be in solution in the form of a dissolved complex, for example a complex with lactoferrin and/or osteopontin. The combination of lactoferrin, osteopontin and iron may be performed by preparing an aqueous solution of lactoferrin and osteopontin and then combining this with iron in aqueous solution. Lactoferrin is typically supplied with some iron complexed to it. This lactoferrin (complexed with iron) may be combined in aqueous solution with osteopontin to form a lactoferrin-osteopontin-iron complex and then a solution of further iron added to increase the amount of iron in the lactoferrin-osteopontin-iron complex. The lactoferrin-osteopontin-iron complex may be formed by taking an aqueous solution of lactoferrin, already complexed with iron, and combining it with an aqueous solution of osteopontin, already complexed with iron.

The lactoferrin, osteopontin and iron may be combined in the process of the invention at a ratio of lactoferrin to osteopontin of between 8:1 and 1:8 by weight. As discussed above, due to the 3:1, 2:1 and 1:1 molar binding stoichiometries at which lactoferrin forms complexes with osteopontin, the ratio of lactoferrin to osteopontin which is combined in the process may not be the same as the ratio in the resulting complex. The term "combined at a ratio of" may refer to the mixing together of components at the stated ratio. To maximize formation of complexes with high levels of osteopontin it may be desirable to limit the amount of lactoferrin combined with osteopontin, for example the lactoferrin, osteopontin and iron may be combined in the process of the invention at a ratio of lactoferrin to osteopontin of between 5:1 and 1:5 by weight. For further example the lactoferrin, osteopontin and iron may be combined in the process of the invention at a ratio of lactoferrin to osteopontin of between 3:1 and 1:3 by weight. To form the complex with the highest level of osteopontin (the 1:1 molar complex) it may be desirable to combine a molar excess of osteopontin with lactoferrin. For example, the lactoferrin, osteopontin and iron may be combined in the process of the invention at a ratio of lactoferrin to osteopontin of between 2:1 and 1:2 by weight.

The lactoferrin-osteopontin-iron complex added to the food product may be in the form of a solution dissolved in water. The complex may be added to the food product as a powder. The process of the invention may further comprise drying the lactoferrin-osteopontin-iron complex to form a powder. For example, the lactoferrin-osteopontin-iron complex may be dried before being added to the food product. Techniques for drying the lactoferrin-osteopontin-iron complex to form a powder are any of those well known in the art. For example, the lactoferrin-osteopontin-iron complex may be separated from aqueous solution by centrifugation and/or filtration and then dried to a powder by freeze drying. Alternatively, an aqueous solution of lactoferrin-osteopontin-iron complex may be spray-dried, optionally together with a carrier such as maltodextrin.

A further aspect of the invention is the use of a lactoferrin-osteopontin-iron complex to fortify a food product with iron wherein the ratio of lactoferrin to osteopontin in the complex is between 6.6:1 and 2.2:1 by weight, for example between 4.4:1 and 2.2:1 by weight, for further example between 3:1 and 2.2:1 by weight. Fortifying food products with a lactoferrin-osteopontin-iron complex is beneficial due to the good bioaccessibility of iron from a lactoferrin-osteopontin-iron complex. A further advantage is that, unlike many other sources of soluble iron, the lactoferrin-osteopontin-iron complex does not lead to significant colour change on heat treatment or during storage.

Foods containing fruit are particularly susceptible to colour change when fortified by iron. It is therefore particularly advantageous that the lactoferrin-osteopontin-iron complex of the invention may be used to fortify a food product comprising fruit. Fruit naturally contains vitamin C, and this vitamin aids in the absorption of iron. The food product comprising fruit according to the invention may comprise fruit at a level of at least 1 wt.% in the food, for example at a level of at least 2 wt.%, for further example at a level of at least 5 wt.%. The maximum level of fruit may be close to 100 wt.%, for example a fruit purée fortified by lactoferrin-osteopontin-iron complex at 0.015% would contain 99.985 wt.% fruit if there were no other ingredients. For processed fruits such as dried fruit or fruit powder, 1 wt.% means 1% by weight of fresh fruit equivalent. The fruit may, for example, be in the form of fresh fruit, fresh fruit pieces, fruit powder, dried fruit, or fruit purée. Fruit provides beneficial dietary nutrients, such as vitamins and minerals together with dietary antioxidants such as polyphenols. These strong nutritional credentials make food compositions comprising fruit a suitable vehicle for further fortification, such as with iron. Fruit can also add attractive texture and colour to food compositions. The fruit may be selected from the group consisting of strawberry, raspberry, blueberry, blackberry, apricot, pear, banana, quince, wolfberry and mixtures of these.

The lactoferrin-osteopontin-iron complex of the invention may be used to fortify a food product wherein the food product is a fruit purée or fruit yoghurt. It is beneficial to be able to fortify fruit purees and fruit yoghurts with vitamins and minerals. Fruit purees and fruit yoghurts are suitable foods for infants and young children, with fruit purees commonly introduced to infants' diets from the age of 6-7 months and fruit yoghurts from 8-12 months. It is important for infants and young children to eat a balanced diet including foods rich in iron. However, fortifying fruit purees and fruit yoghurts with iron may make them sensitive to undesirable colour changes. It is therefore advantageous that the use of a lactoferrin-osteopontin-iron complex according to the current invention allows the fortification of fruit purees and fruit yoghurts with iron, whilst preventing or reducing colour change.

Those skilled in the art will understand that they can freely combine all features of the present invention disclosed herein. In particular, features described for the product of the present invention may be combined with the process of the present invention and vice versa. Further, features described for different embodiments of the present invention may be combined. Where known equivalents exist to specific features, such equivalents are incorporated as if specifically referred to in this specification. Further advantages and features of the present invention are apparent from the figure and non-limiting examples.

Example 1: Preparation of lactoferrin-osteopontin-iron complex with high proportion of osteopontin.

500 mg of lactoferrin (Armor proteins) was dissolved in 5 ml Millipore water and 500 mg of osteopontin (Lacprodan OPN-10, Arla) was dissolved in a further 5 ml Millipore water. The two solutions were combined in a 15 ml tube, shaken, and allowed to stand for 15 min. A solution of iron was prepared by weighing 0.3192 g of $FeSO_4 x7H_2O$ (Merck ) into a 15 ml tube, adding 10 ml Millipore water and mixing well. 200 $\mu$l of the iron solution (1280 $\mu$g Fe) was added to the mixture of lactoferrin and osteopontin and allowed to stand for 15 minutes.

Two Amicon Ultra 15 ultra filtration tubes were weighed, with and without an inserted filter. The mixture of lactoferrin, osteopontin and iron was split, pipetting 5 ml into each of the two Amicon Ultra 15 ultra filtration tubes. The tubes were centrifuged for 3h at 4000 g until all the liquid has passed through the filter. Each filter was removed and rinsed with 2-3 ml of Millipore water, the recovered protein being pipetted into a 50 ml Sarstedt tube. The Amicon tubes without filter were weighed to establish the weight of the filtrate. The filtrate was stored in a fridge. The recovered complex was freeze-dried. Approximately 850 mg powder was obtained.

The Fe content of the freeze-dried complex was determined by Inductively Coupled Plasma Atomic Emission Spectroscopy (ICP-AES). The Fe content was 1.425 mg Fe /g powder. The presence of the lactoferrin-osteopontin-iron complex was confirmed by size exclusion chromatography using HPLC-ICPMS (High-Performance Liquid Chromatography with Inductively Coupled Plasma Mass Spectrometry).

Lactoferrin, osteopontin and iron were combined at a ratio of lactoferrin to osteopontin of 1:1 by weight, this is a molar ratio of 1:2.22. The excess of osteopontin ensures that a lactoferrin-osteopontin-iron complex with a 1:1 molar ratio of lactoferrin to osteopontin will be formed. This is a complex having a ratio of lactoferrin to osteopontin in the complex of 2.2:1 by weight. The freeze-dried powder therefore contains 72.5 % lactoferrin-osteopontin-iron complex by weight.

Example 2: Preparation of lactoferrin-osteopontin-iron complex with high proportion of lactoferrin.

The procedure of example 1 was repeated, except that 1000 mg of lactoferrin dissolved in 5ml of Millipore water was combined with 100 mg of osteopontin dissolved in 5 ml Millipore water. Approximately 935 mg of powder was obtained. The Fe content was approximately 1.5 mg Fe /g powder. The presence of the lactoferrin-osteopontin-iron complex was confirmed by size exclusion chromatography using HPLC-ICPMS.

In example 2, lactoferrin, osteopontin and iron were combined at a ratio of lactoferrin to osteopontin of 10:1 by weight; this is a molar ratio of 4.5:1. The excess of lactoferrin ensured that a lactoferrin-osteopontin-iron complex with a 3:1 molar ratio of lactoferrin to osteopontin was formed. This is a complex having a ratio of lactoferrin to osteopontin in the complex of 6.6:1 by weight. The freeze-dried powder therefore contains 76 % lactoferrin-osteopontin-iron complex by weight.

Example 3: Iron fortified strawberry-banana yoghurt

Commercial yoghurt, Nestle Jogolino™ Strawberry/Banana yoghurt containing 15 % banana puree and 10 % strawberry puree, was iron fortified by the addition of different iron containing materials to 5 kg yoghurt as reported in the table below. The amounts were chosen to provide approximately 0.8 mg iron per 100g yoghurt. A 100g serving of yoghurt with that level of iron would provide 15% of the recommended daily amount of iron for an infant [Official Journal of the European Union, Commission Directive 2006/125/EC].

Ferrous sulphate and ferric sodium EDTA were obtained from *Dr Paul Lohmann™*. Lactoferrin was obtained from Armor proteins.

For the lactoferrin-osteopontin-iron complex (Trial B), the powder from Example 1 was added to yoghurt at a level of 28g per 5kg yoghurt. The resulting yoghurt composition comprised 5.6g powder per kg which is 4g LF-OPN-Fe complex per kg.

The yoghurts were flash pasteurized at 105 °C for 2 minutes. Colour measurements were performed in 1x1 cm polystyrene cuvettes using an X-Rite ColorEye 7000A colorimeter. The colorimeter was set up with a D65 light source, 10 degree observer angle and with specular component included. The colour difference between the yoghurt with no iron salts and the iron fortified yoghurt was measured for each iron salt and expressed as $\Delta$Eab* using the CIELAB colour scale.

| Trial | Fe material | % Fe | Amount (g) | $\Delta$Eab* |
|---|---|---|---|---|
| A | $FeSO_4 xH_2O$ | 32.0 | 0.1243 | 4.74 |
| B | LF-OPN-Fe (powder from Example 1) | 0.14 | 28 | 2.11 |

(continued)

| Trial | Fe material | % Fe | Amount (g) | ΔEab* |
|-------|-------------|------|-----------|-------|
| C | Lactoferrin-Fe | 0.0151 | 262.5 | 5.64 |
| D | NaFeEDTA x $H_2O$ | 12.5 | 0.318 | 3.11 |

The yoghurt fortified with lactoferin-osteopontin-iron complex showed the smallest colour change on pasteurization.

Example 4: Iron bio-accessibility of iron fortified strawberry-banana yoghurts

The bio-accessibility of iron in the yoghurts fortified with different iron-containing compounds was measured using Caco-2 cells in conjunction with in vitro digestion [R.P. Glahn et al., Journal of Food Science, 64(5), 925-928. (1999)]. The level of iron in all the yoghurts was 100μg/g.

In brief, first the yoghurt was subjected to a simulated gastric digestion with pepsin at pH=2, 37°C for 1 hour. This step was followed by a simulated intestinal digestion with pancreatin and bile at pH=7, 37°C for 2 hours. This second step took place on a dialysis membrane in the presence of Caco-2 monolayers. During the digestion process iron is released from the food matrix. Then, solubilized Fe can diffuse and be taken up by the cells. Thus, in response to higher intracellular Fe concentrations, Caco-2 cell will form ferritin. Therefore, the formation of ferritin is quantified as an indicator of Fe uptake by the cells. Ferritin is measured by ELISA in harvested Caco-2 cell 24 hours after the digestion. Results are normalized with the total protein contents of Caco2 cells and are expressed as ng ferritin/mg protein. To ensure the robustness of the results, three experiments were performed on separate days using cells from the same batches with three replicates. Data collected from the same treatment were pooled. Enzymes and buffer are used as blank. NaFeEDTA fortified at 100 μg/g with Fe was used as an internal standard and as positive control (D). The yoghurts fortified with lactoferin-osteopontin-iron complex (B) were found to have a similar bioaccessibility to those fortified with $FeSO_4$ (A), see figure 1.

**Claims**

1.    A composition comprising at least 2 g/kg of lactoferrin-osteopontin-iron complex for use in the treatment or prevention of iron deficiency.

2.    Composition for use according to claim 1 wherein the ratio of lactoferrin to osteopontin in the complex is between 6.6:1 and 2.2:1 by weight.

3.    Composition for use in accordance with claim 1 or claim 2 to be administered orally or enterally.

4.    Composition for use according to any one of claims 1 to 3 to be administered to preterm or low birth weight infants.

5.    Composition for use according to any one of claims 1 to 3 to be administered to pregnant or lactating women.

6.    A fortified foodstuff comprising at least 2 g/kg of lactoferrin-osteopontin-iron complex.

7.    Fortified foodstuff according to claim 6 wherein the ratio of lactoferrin to osteopontin in the complex is between 6.6:1 and 2.2:1 by weight.

8.    Fortified foodstuff according to claim 6 or claim 7 wherein the foodstuff is a dairy product; a culinary product; a food for infants; a food for pregnant women or new mothers, a beverage; a biscuit, cake or pastry product; a dessert; a nutritional formula or a pet food product.

9.    Fortified foodstuff according to any one of claims 6 to 8 wherein the colour change of the fortified foodstuff measured as ΔEab* is less than 3.5 after a heat treatment of 2 minutes at 105 °C.

10.   A process for fortifying a food product with iron comprising forming a lactoferrin-osteopontin-iron complex by combining lactoferrin, osteopontin and iron, the lactoferrin, osteopontin and iron being in aqueous solution; preparing a food product; and adding the lactoferrin-osteopontin-iron complex to the food product.

11. Process according to claim 10 wherein the lactoferrin, osteopontin and iron are combined at a ratio of lactoferrin to osteopontin of between 8:1 and 1:8 by weight.

12. Process according to claim 10 or claim 11 further comprising drying the lactoferrin-osteopontin-iron complex to form a powder.

13. Use of a lactoferrin-osteopontin-iron complex to fortify a food product with iron wherein the ratio of lactoferrin to osteopontin in the complex is between 6.6:1 and 2.2:1 by weight.

14. Use according to claim 13 wherein the food product comprises fruit.

15. Use of according to claim 13 or claim 14 wherein the food product is a fruit purée or fruit yoghurt.


**Patentansprüche**

1. Zusammensetzung, die mindestens 2 g/kg Lactoferrin-Osteopontin-Eisen-Komplex umfasst, zur Verwendung bei der Behandlung oder Prävention von Eisenmangel.

2. Zusammensetzung zur Verwendung nach Anspruch 1, wobei das Gewichtsverhältnis von Lactoferrin zu Osteopontin im Komplex zwischen 6,6:1 und 2,2:1 beträgt.

3. Zusammensetzung zur Verwendung nach Anspruch 1 oder Anspruch 2, die entweder oral oder enteral zu verabreichen ist.

4. Zusammensetzung zur Verwendung nach einem der Ansprüche 1 bis 3, die an Frühgeburten oder Säuglinge mit niedrigem Geburtsgewicht zu verabreichen ist.

5. Zusammensetzung zur Verwendung nach einem der Ansprüche 1 bis 3, die an schwangere oder stillende Frauen zu verabreichen ist.

6. Angereichertes Nahrungsmittel, das mindestens 2 g/kg Lactoferrin-Osteopontin-Eisen-Komplex umfasst.

7. Angereichertes Nahrungsmittel zur Verwendung nach Anspruch 6, wobei das Gewichtsverhältnis von Lactoferrin zu Osteopontin im Komplex zwischen 6,6:1 und 2,2:1 beträgt.

8. Angereichertes Nahrungsmittel nach Anspruch 6 oder Anspruch 7, wobei es sich bei dem Nahrungsmittel um ein Milchprodukt, ein kulinarisches Produkt, eine Säuglingsnahrung, ein Nahrungsmittel für schwangere Frauen oder Wöchnerinnen, ein Getränk, einen Keks, einen Kuchen oder ein Gebäckprodukt, ein Dessert, eine Nährstoffrezeptur oder ein Tiernahrungsprodukt handelt.

9. Angereichertes Nahrungsmittel nach einem der Ansprüche 6 bis 8, wobei der Farbwechsel des angereicherten Nahrungsmittels gemessen als ΔEab* nach einer Wärmebehandlung von 2 Minuten bei 105 °C weniger als 3,5 beträgt.

10. Verfahren zum Anreichern eines Nahrungsmittelprodukts mit Eisen, umfassend das Formulieren eines Lactoferrin-Osteopontin-Eisen-Komplexes durch Kombinieren von Lactoferrin, Osteopontin und Eisen, wobei sich Lactoferrin, Osteopontin und Eisen in wässriger Lösung befinden; Herstellen eines Nahrungsmittelprodukts; und Hinzufügen des Lactoferrin-Osteopontin-Eisen-Komplexes zum Nahrungsmittelprodukt.

11. Verfahren nach Anspruch 10, wobei das Lactoferrin, das Osteopontin und das Eisen in einem Gewichtsverhältnis von Lactoferrin zu Osteopontin zwischen 8:1 und 1:8 kombiniert werden.

12. Verfahren nach Anspruch 10 oder Anspruch 11, das ferner das Trocknen des Lactoferrin-Osteopontin-Eisen-Komplexes zur Bildung eines Pulvers umfasst.

**13.** Verwendung eines Lactoferrin-Osteopontin-Eisen-Komplexes zum Anreichern eines Nahrungsmittelprodukts mit Eisen, wobei das Gewichtsverhältnis von Lactoferrin zu Osteopontin im Komplex zwischen 6,6:1 und 2,2:1 beträgt.

**14.** Verwendung nach Anspruch 13, wobei das Nahrungsmittelprodukt Früchte umfasst.

**15.** Verwendung nach Anspruch 13 oder Anspruch 14, wobei es sich bei dem Nahrungsmittelprodukt um Fruchtmus oder Fruchtjoghurt handelt.

**Revendications**

**1.** Composition comprenant au moins 2 g/kg de complexe lactoferrine-ostéopontine-fer à utiliser dans le traitement ou la prévention d'une carence en fer.

**2.** Composition à utiliser selon la revendication 1, dans laquelle le rapport de la lactoferrine à l'ostéopontine dans le complexe est entre 6,6:1 et 2,2:1 en poids.

**3.** Composition à utiliser selon la revendication 1 ou la revendication 2 à administrer par voie orale ou entérale.

**4.** Composition à utiliser selon l'une quelconque des revendications 1 à 3 à administrer à des nourrissons prématurés ou à faible poids à la naissance.

**5.** Composition à utiliser selon l'une quelconque des revendications 1 à 3 à administrer à des femmes enceintes ou qui allaitent.

**6.** Produit alimentaire enrichi comprenant au moins 2 g/kg de complexe lactoferrine-ostéopontine-fer.

**7.** Produit alimentaire enrichi selon la revendication 6, dans lequel le rapport de la lactoferrine à l'ostéopontine dans le complexe est de 6,6:1 à 2,2:1 en poids.

**8.** Produit alimentaire enrichi selon la revendication 6 ou la revendication 7, dans lequel le produit alimentaire est un produit laitier ; un produit culinaire ; un aliment pour nourrissons ; un aliment pour femmes enceintes ou jeunes mères, une boisson ; un biscuit, un gâteau ou un produit de pâtisserie ; un dessert ; une formule nutritionnelle ou un produit alimentaire pour animal de compagnie.

**9.** Produit alimentaire enrichi selon l'une quelconque des revendications 6 à 8, dans lequel le changement de couleur du produit alimentaire enrichi mesuré en $\Delta$Eab* est inférieur à 3,5 après un traitement thermique de 2 minutes à 105 °C.

**10.** Processus d'enrichissement d'un produit alimentaire en fer comprenant la formation d'un complexe lactoferrine-ostéopontine-fer par combinaison de lactoferrine, d'ostéopontine et de fer, la lactoferrine, l'ostéopontine et le fer étant en solution aqueuse ; la préparation d'un produit alimentaire ; et l'ajout du complexe lactoferrine-ostéopontine-fer au produit alimentaire.

**11.** Processus selon la revendication 10, dans lequel la lactoferrine, l'ostéopontine et le fer sont combinés à un rapport de lactoferrine à l'ostéopontine allant de 8:1 à 1:8 en poids.

**12.** Processus selon la revendication 10 ou la revendication 11 comprenant en outre le séchage du complexe lactoferrine-ostéopontine-fer afin de former une poudre.

**13.** Utilisation d'un complexe lactoferrine-ostéopontine-fer afin d'enrichir un produit alimentaire en fer, dans laquelle le rapport de la lactoferrine à l'ostéopontine dans le complexe va de 6,6:1 à 2,2:1 en poids.

**14.** Utilisation selon la revendication 13, dans laquelle le produit alimentaire comprend du fruit.

**15.** Utilisation selon la revendication 13 ou la revendication 14, dans laquelle le produit alimentaire est une purée de fruits ou un yaourt aux fruits.

**Fig. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1011344 A
- WO 9715201 A
- EP 1040766 A
- WO 2005051088 A
- WO 2004060392 A

**Non-patent literature cited in the description**

- **J. UMBREIT.** *American Journal of Hematology,* 2005, vol. 78, 225
- Guidelines on food fortification with micronutrients. World Health Organization, 2006
- **A.P.YAMNIUK et al.** *Molecular Immunology,* 2009, vol. 46, 2395
- **R.J. MILLS et al.** Enteral iron supplementation in preterm and low birth weight infants. *Cochrane database of systematic reviews,* CD005095
- CIE Technical Report. 1996
- **R.P. GLAHN et al.** *Journal of Food Science,* 1999, vol. 64 (5), 925-928